(19) **Europäisches Patentamt**
**European Patent Office**
**Office européen des brevets**

(11) **EP 4 114 257 B1**

(12) **EUROPEAN PATENT SPECIFICATION**

(45) Date of publication and mention
of the grant of the patent:
**04.06.2025 Bulletin 2025/23**

(21) Application number: **21715963.1**

(22) Date of filing: **04.03.2021**

(51) International Patent Classification (IPC):
*A61B 5/024* (2006.01)    *A61B 5/00* (2006.01)
*A61B 5/021* (2006.01)    *A61B 5/02* (2006.01)

(52) Cooperative Patent Classification (CPC):
**A61B 5/02405; A61B 5/02028; A61B 5/021;**
**A61B 5/4035; A61B 5/4884;** A61B 2562/0247

(86) International application number:
**PCT/IB2021/051831**

(87) International publication number:
**WO 2021/176399 (10.09.2021 Gazette 2021/36)**

(54) **METHOD OF DETECTING PARAMETERS INDICATIVE OF ACTIVATION OF SYMPATHETIC AND PARASYMPATHETIC NERVOUS SYSTEMS**

VERFAHREN ZUR ERKENNUNG VON PARAMETERN, DIE AUF DIE AKTIVIERUNG SYMPATHISCHER UND PARASYMPATHISCHER NERVENSYSTEME HINWEISEN

PROCÉDÉ DE DÉTECTION DE PARAMÈTRES INDICATIFS DE L'ACTIVATION DE SYSTÈMES NERVEUX SYMPATHIQUE ET PARASYMPATHIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.03.2020 IT 202000004621**

(43) Date of publication of application:
**11.01.2023 Bulletin 2023/02**

(73) Proprietor: **Romano, Salvatore**
**50133 Firenze (FI) (IT)**

(72) Inventor: **Romano, Salvatore**
**50133 Firenze (FI) (IT)**

(74) Representative: **IP Sextant s.r.l.**
**Via A. Salandra, n.18**
**00187 Rome (IT)**

(56) References cited:
• **CHENGYU LIU ET AL: "Systolic and Diastolic Time Interval Variability Analysis and Their Relations with Heart Rate Variability", BIOINFORMATICS AND BIOMEDICAL ENGINEERING , 2009. ICBBE 2009. 3RD INTERNATIONAL CONFERENCE ON, IEEE, PISCATAWAY, NJ, USA, 11 June 2009 (2009-06-11), pages 1 - 4, XP031489349, ISBN: 978-1-4244-2901-1**
• **PARK JI HYUN ET AL: "Respiratory variation of systolic and diastolic time intervals within radial arterial waveform: a comparison with dynamic preload index", JOURNAL OF CLINICAL ANESTHESIA, BUTTERWORTH PUBLISHERS, STONEHAM, GB, vol. 32, 24 March 2016 (2016-03-24), pages 75 - 81, XP029596121, ISSN: 0952-8180, DOI: 10.1016/ J.JCLINANE.2015.12.022**
• **FAUSTO LUCENA ET AL: "Heart Instantaneous Frequency Based Estimation of HRV from Blood Pressure Waveforms", IEICE TRANSACTIONS ON INFORMATION AND SYSTEMS, vol. E92-D, no. 3, 1 March 2009 (2009-03-01), JP, pages 529 - 537, XP055741975, ISSN: 0916-8532, DOI: 10.1587/transinf.E92.D.529**

**EP 4 114 257 B1**

**(Cont. next page)**

- **HE'BERT ET AL: "Relation between aortic dicrotic notch pressure and mean aortic pressure in adults", AMERICAN JOURNAL OF CARDIOLOGY, CAHNERS PUBLISHING CO., NEWTON, MA, US, vol. 76, no. 4, 1 August 1995 (1995-08-01), pages 301 - 306, XP005497327, ISSN: 0002-9149, DOI: 10.1016/S0002-9149(99) 80086-1**

**EP 4 114 257 B1**

**Description**

The present invention concerns a computer-implemented method for detecting parameters indicative of a variation of activation of the sympathetic nervous system and of a variation of activation of the parasympathetic nervous system, from which it is also possible to evaluate a variation in the balance between the activity of the sympathetic nervous system and the activity of the parasympathetic nervous system, in a subject in the transition from a basic condition (hereinafter also referred to as a basal condition) to a perturbed condition, thereby such method provides an indication for discriminating between an adequate balance and an imbalance between the activation of the sympathetic nervous system and the activation of the parasympathetic nervous system of the subject in the transition from the basal condition to the perturbed condition. The computer-implemented method is capable, in a simple, versatile, efficient and reliable way, to indicate the effect of the transition of the subject himself/herself from the basal condition to the perturbed condition on the interaction between such sympathetic and parasympathetic nervous systems, for example to determine the effect on such interaction of the application of a drug and/or the change in posture of the subject himself/herself.

The present invention also concerns an apparatus configured to perform such method.

The method according to the invention is a computer-implemented method, where the term "computer" means any processing device (in particular, at least one microprocessor), which executes a set of one or more computer programs comprising instructions which, when executed by an apparatus according to the invention, cause the same apparatus to perform the computer-implemented method for detecting the activation of the vagal system. Also, said one or more computer programs can be stored on a set of one or more computer-readable media.

It is known that heart rate can be defined as the average number of heartbeats per minute. This number, for example 70 beats per minute (b/m), is an average value, because the time between one heartbeat and the next is actually not constant and changes continuously. The Heart Rate Variability, also known as HRV, is a useful parameter for assessing a subject's health. In fact, the measurement and analysis of HRV are assuming an increasing importance as from this measurement it is possible to deduce a lot of information, allowing for example to assess the risk of cardiac arrhythmias and heart attack, as well as whether the balance between the activity of the system orthosympathetic nervous system, also known as sympathetic nervous system, and the activity of the parasympathetic nervous system is correct or not. In this regard, although the evaluation of HRV originated limitedly to the field of cardiology, numerous recent scientific studies have shown its importance as a reliable indicator also in numerous other application fields.

It is known that the HRV is the natural variability of the heart rate in response to factors such as breathing rhythm, emotional states such as anxiety, stress, anger, relaxation. In a healthy heart, the heart rate responds quickly to all these factors, changing according to the situation, to better adapt the body to the different conditions it undergoes. In general, a healthy subject shows a good degree of the heart rate variability, i.e. an adequate degree of psychophysical adaptability to different situations.

The HRV is correlated to the interaction between the sympathetic nervous system and the parasympathetic nervous system, which in turn affect functioning of organs and systems of the body, such as cardiovascular and respiratory interaction.

The sympathetic nervous system, when activated, produces a series of effects such as: acceleration of the heartbeat, dilation of the bronchi, increase in blood pressure, peripheral vasoconstriction, pupillary dilation, increased sweating. The chemical mediators of these vegetative responses are norepinephrine, adrenaline, corticotropin, and several corticosteroids. The sympathetic nervous system is the body's normal response to a situation of alarm, struggle, physical and/or emotional stress (also known as the "fight or flight" response).

Conversely, the parasympathetic nervous system (that also expresses through the vagal tone, i.e. the activity of the vagus nerve or vagal activity), when activated, produces a slowing of the heart rhythm, an increase in bronchial muscle tone, dilation of the blood vessels, decrease in pressure, slowed breathing, increased muscle relaxation, breathing becomes calmer and deeper, genitals, hands and feet become warmer. It acts through the typical chemical mediator acetylcholine. The parasympathetic nervous system represents the body's normal response to a situation of calm, rest, tranquillity and the absence of dangers and (physical and emotional) stress.

The organism of a subject, at any moment, is in a situation determined by the balance or the predominance of one of these two nervous systems (i.e. the sympathetic nervous system and the parasympathetic nervous system). The ability of the organism to change its own balance through a greater activation of one or the other nervous system is very important and is a fundamental mechanism tending to the dynamic balance of the organism both from a physiological and psychological point of view.

The evaluation of the HRV allows to evaluate the relative balance state between the activation of the sympathetic nervous system and the activation of the parasympathetic nervous system. This is of great importance for assessing when and how these two systems reach the best balance in specific situations and/or in specific types of patients (who can be both healthy and pathological subjects).

Generally the HRV is evaluated by measurements made through an electrocardiographic machine, also known as an ECG or EKG, provided with conventional surface electrodes which are applied at the level of the heart to detect the electrical activity of the heart (e.g., see J. W. Hurst, "Naming of the Waves in the ECG, With a Brief Account of Their Genesis" in Circulation, vol. 98, nº 18, 3 November 1998, pp. 1937-42), in which a related very complex dedicated software performs the analysis of data by identifying the individual beats and thus their variability. By way of example, and not by way of limitation, examples of such softwares are those available from the Italian company Elemaya (see www.elemaya.it) and those available from the Finnish company Kubios Oy (www.kubios.com). In particular, after having been digitized, data are analysed by a computer-implemented method with a software calculating the time distance (usually expressed in milliseconds) between each heartbeat and the next one by measuring the time distance between the R peaks of the ECG signal, then building a diagram, called tachogram, that represents the trend of the RR distance between one beat and the next one (ordinate axis), usually expressed in milliseconds, as a function of the progressive number of the heartbeats (abscissa axis). The tachogram is usually made for a time interval of 4-5 minutes (i.e. for a total number of about 300 heartbeats).

Subsequently, the software performs a resampling of the tachogram and subsequently the Fourier transform to obtain the power spectrum, i.e. the power spectral density, also indicated as PSD of the tachogram resulting from the resampling operation (e.g., see J. Pucik et al. in "Heart Rate Variability Spectrum: Physiologic Aliasing and Nonstationarity Considerations", Trends in Biomedical Engineering Conference paper, Bratislava, September 16 - 18, 2009).

The power spectrum PSD represents the frequency components of the tachogram and contains essential information to arrive at an evaluation of the balance between the activation of the sympathetic nervous system and the activation of the parasympathetic nervous system. In particular, the power spectrum PSD of the tachogram expresses the power (in the frequency domain) of the tachogram at frequencies between 0,01 Hz and 0,4 Hz. The power is usually expressed in milliseconds squared.

Studies and researches in recent years (e.g., see A.E. Aubert et al. in "Heart rate variability in athletes", Sports Medicine 33 (12):889-919, 2003), have permitted to distinguish three subbands of frequencies, called respectively:

- VLF (*Very Low Frequency*) band, for frequencies between 0,01 Hz and 0,04 Hz, that depends on changes in thermoregulation and, in the psychological context, is affected by conditions of worry and obsessive thoughts (*worry and rumination*), and it is only marginally due to the activity of the sympathetic nervous system;
- LF (Low Frequency) band for frequencies between 0,04 Hz and 0,15 Hz, that is considered mainly due to the activity of the sympathetic nervous system and to the regulation of baroreceptors; and
- HF (High Frequency) band for frequencies between 0,15 Hz and 0,4 Hz, that is considered an expression of the activity of the parasympathetic nervous system and, hence, of its fundamental component constituted by the vagal activity; in particular, the HF band is strongly affected by the rhythm and depth of respiration, whereby altered rhythm and/or depth of respiration rise the contribution of the HF band to the power spectrum PSD of the tachogram.

The relationship between the activity of the sympathetic nervous system and the activity of the parasympathetic nervous system is evaluated through the LF/HF ratio between the power of the tachogram in the LF band and the power of the tachogram in the HF band (possibly normalized to their sum). In particular, in literature the power values are often also expressed in their logarithmic form.

Finally, the software can also calculate the standard deviation SD and/or the total power of the tachogram (possibly in logarithmic form), where the total power is commonly set equal to the square of the standard deviation of the tachogram (e.g., see A.E. Aubert et al., cited above). Both of these parameters express the overall degree of the HRV, thus the overall activity of the sympathetic nervous system and parasympathetic nervous system.

Further studies in this context, related to a correlation between analysis of the variability of systolic and diastolic time intervals on the basis of ECG and phonocardiogram signals (PCG - Phonocardiogram) and the HRV for an evaluation of the cardiovascular nonlinear dynamics were carried out by Chengyu Liu et al. in "Systolic and Diastolic Time Interval Variability Analysis and Their Relations with Heart Rate Variability", BIOINFORMATICS AND BIOMEDICAL ENGINEERING, 2009, ICBBE 2009. 3RD INTERNATIONAL CONFERENCE ON, IEEE, PISCATAWAY, NJ, USA, 11 June 2009 (2009-06-11), pages 1-4, XP031489349, ISBN: 978-1-4244-2901-1. Also, a study on the possible comparison of respiratory variations of the systolic and diastolic time intervals within the radial arterial waveform with dynamic indices was carried out by Park Ji Hyun et al. in "Respiratory variation of systolic and diastolic time intervals within radial arterial waveform: a comparison with dynamic preload index", JOURNAL OF CLINICAL ANESTHESIA, BUTTERWORTH PUBLISHERS, STONEHAM, GB, vol. 32, 24 March 2016 (2016-03-24), pages 75-81, XP029596121, ISSN: 0952-8180, DOI: 10.1016 / J.JCLINANE.2015.12.022.

Clinical experience in recent years has also permitted to define reference ranges for the values of the parameters mentioned above, namely of heart rate, tachogram standard deviation SD, tachogram total power, tachogram power in the VLF band, tachogram power in the LF band and tachogram power in the HF band. Although the definition of the reference ranges is not completely equal between different authors and between the American and European standards, in the context of prior art softwares reference ranges have been adopted which are derived from an experimental basis related to the population under consideration (e.g., the Italian population in the case of studies and researches carried out on Italian subjects).

Moreover, different reference ranges have also been introduced in relation to an elderly population (from 50 to 70 years of age) or to a young population (from 20 to 50 years of age).

However, prior art methods of evaluating the state of balance between the activity of the sympathetic nervous system and the activity of the parasympathetic nervous system, based on the evaluation of the HRV, still suffer from a lack of uniformity of interpretation of the results obtainable from the analysis of the measurements carried out. By way of example, in literature there are widely different, if not conflicting, indications in relation to the time intervals in which the analysis have to be performed (i.e. collecting data to build the tachogram to be analysed), and to the pathologies of the subjects examined to which the results obtained from the analysis of measurements made on a single subject have to be referred.

The object of the present invention is therefore to allow to evaluate in a simple, versatile, efficient and reliable way, the activation of the sympathetic nervous system and the parasympathetic nervous system, as well as the balance between the activity of the sympathetic nervous system and the activity of the parasympathetic nervous system, thus permitting to indicate the effect of the transition of the subject himself/herself from a basal condition to a perturbed condition on the interaction between such sympathetic and parasympathetic nervous systems, for example to determine the effect on such interaction of the application of a drug and/or the change in posture of the subject himself/herself.

It is a specific object of the present invention a computer-implemented method of detecting parameters indicative of a variation of activation of sympathetic nervous system and of a variation of activation of parasympathetic nervous system in a subject in a transition from a basal condition to a perturbed condition, comprising the following steps:

A. receiving a discrete pressure signal $p(t_i)$ of the subject comprising a plurality of heartbeats;

B. identifying each heartbeat of the discrete pressure signal $p(t_i)$ and, within each heartbeat, identifying a systolic phase $p_{sys}(t_i)$ and a diastolic phase $p_{dia}(t_i)$;

C. building a diagram $D_{sys}$ of duration of the systolic phase as a function of a heartbeat progressive number and a diagram $D_{dia}$ of duration of the diastolic phase as a function of the heartbeat progressive number;

D. executing a resampling of the diagram $D_{sys}$ of duration of the systolic phase, obtaining a resampled diagram $D_{sys}^{(r)}$ of duration of the systolic phase, and a resampling of the diagram $D_{dia}$ of duration of the diastolic phase, obtaining a resampled diagram $D_{dia}^{(r)}$ of duration of the diastolic phase;

E. calculating a power spectrum $PSD_{sys}$ of the resampled diagram $D_{sys}^{(r)}$ of duration of the systolic phase and a power spectrum $PSD_{dia}$ of the resampled diagram $D_{dia}^{(r)}$ of duration of the diastolic phase at frequencies between a lower limit frequency $f_{lower\text{-}limit}$ and a upper limit frequency $f_{upper\_limit}$ higher than the lower limit frequency $f_{lower\_limit}$;

F. calculating a power $P_{LF}^{(PSD_{sys})}$ of the power spectrum $PSD_{sys}$ in a LF band, a power $P_{HF}^{(PSD_{sys})}$ of the power spectrum $PSD_{sys}$ in a HF band, a power $P_{LF}^{(PSD_{dia})}$ in the LF band of the power spectrum $PSD_{dia}$, and a power $P_{HF}^{(PSD_{dia})}$ in the HF band of the power spectrum $PSD_{dia}$, wherein the frequency $f_{LF}$ in the LF band is higher than or equal to a first intermediate frequency $f_{intermediate\_1}$ and lower than a second intermediate frequency $f_{intermediate\_2}$, thereby

$$f_{intermediate\_1} \le f_{LF} < f_{intermediate\_2},$$

wherein the lower limit frequency $f_{lower\_limit}$ is lower than the first intermediate frequency $f_{intermediate\_1}$. that is in turn lower than the second intermediate frequency $f_{intermediate\_2}$, that is in turn lower than the upper limit frequency $f_{upper\_limit}$, thereby

$$f_{lower\_limit} < f_{intermediate\_1} < f_{intermediate\_2} < f_{upper\_limit},$$

and wherein the frequency $f_{HF}$ in the HF band is higher than or equal to the second intermediate frequency $f_{intermediate\_2}$ and lower than the upper limit frequency $f_{upper\_limit}$, thereby

$$f_{intermediate\_2} \leq f_{HF} < f_{upper\_limit} \;;$$

and

G. calculating and outputting a value of a ratio $LHR_{sys}$ between the powers in the LF and HF bands of the power spectrum $PSD_{sys}$ and a value of a ratio $LHR_{dia}$ between the powers in the LF and HF bands of the power spectrum $PSD_{dia}$, thereby

$$LHR_{sys} = \frac{P_{LF}^{(PSD_{sys})}}{P_{HF}^{(PSD_{sys})}}$$

$$LHR_{dia} = \frac{P_{LF}^{(PSD_{dia})}}{P_{HF}^{(PSD_{dia})}}$$

wherein steps A-G of the computer-implemented method are executed on the subject first in a basal condition and then in a perturbed condition.

According to another aspect of the invention, the lower limit frequency $f_{lower\_limit}$ can be equal to 0,01 Hz, the upper limit frequency $f_{upper\_limit}$ can range from 0,4 Hz to 1,2 Hz, the first intermediate frequency $f_{intermediate\_1}$ can range from 0,04 Hz to 0,12 Hz, and the second intermediate frequency $f_{intermediate\_2}$ can range from 0,15 Hz to 0,45 Hz, wherein optionally the upper limit frequency $f_{upper\_limit}$ can range from 0,8 Hz to 1,2 Hz, the first intermediate frequency $f_{intermediate\_1}$ can range from 0,08 Hz to 0,12 Hz, and the second intermediate frequency $f_{intermediate\_2}$ can range from 0,30 Hz to 0,45 Hz, wherein more optionally the upper limit frequency $f_{upper\_limit}$ can be equal to 1,2 Hz, the first intermediate frequency $f_{intermediate\_1}$ can be equal to 0,12 Hz, and the second intermediate frequency $f_{intermediate\_2}$ can be equal to 0,45 Hz.

According to a further aspect of the invention, in step E, the power spectra $PSD_{sys}$ and $PSD_{dia}$ can be calculated through a Fourier transform, optionally through a Fast Fourier Transform (FFT), of the resampled diagram $D_{sys}^{(r)}$ of duration of the systolic phase and of the resampled diagram $D_{dia}^{(r)}$ of duration of the diastolic phase, respectively.

According to an additional aspect of the invention, the discrete pressure signal $p(t_i)$ received in step A can have a time duration of at least 3 minutes, optionally of at least 4 minutes, more optionally of at least 5 minutes.

According to another aspect of the invention, in step B, the systolic phase and the diastolic phase of each heartbeat can be identified on the basis of identification of the dicrotic notch time.

According to a further aspect of the invention, the computer-implemented method can:

- in step B, further identify a value of dicrotic notch pressure $P_{dic}$ in each heartbeat;
- in step C, further build a diagram $D_{dic}$ of dicrotic notch pressure as a function of the heartbeat progressive number;
- in step D, further execute a resampling of the diagram $D_{dic}$ of dicrotic notch pressure obtaining a resampled diagram $D_{dic}^{(r)}$ of dicrotic notch pressure;

- in step E, further calculate a power spectrum $PSD_{dic}$ of the resampled diagram $D_{dic}^{(r)}$ of dicrotic notch pressure at frequencies between the lower limit frequency $f_{lower\_limit}$ and the upper limit frequency $f_{upper\_limit}$;

- in step F, further calculate a power $P_{LF}^{(PSD_{dic})}$ of the power spectrum $PSD_{dic}$ in the LF band and a power $P_{HF}^{(PSD_{dic})}$ of the power spectrum $PSD_{dic}$ in the HF band; and
- in step G, further calculate and output a value of a ratio $LHR_{dic}$ between the powers in the LF and HF bands of the power spectrum $PSD_{dic}$, thereby:

$$LHR_{dic} = \frac{P_{LF}^{(PSD_{dic})}}{P_{HF}^{(PSD_{dic})}}$$

According to an additional aspect of the invention, in step C the diagram $D_{sys}$ of duration of the systolic phase and the diagram $D_{dia}$ of duration of the diastolic phase can be built by expressing the duration of the systolic phase and of the diastolic phase of each heartbeat as value normalised to an overall duration of the heartbeat under consideration.

According to another aspect of the invention, the computer-implemented method can further comprise determining and outputting a HRV (*Heart Rate Variability*) of the subject first in the basal condition and then in the perturbed condition.

According to a further aspect of the invention, the computer-implemented method can further calculate a standard deviation $SD^{(sys)}$ of the resampled diagram $D_{sys}^{(r)}$ of duration of the systolic phase and a standard deviation $SD^{(dia)}$ of the resampled diagram $D_{dia}^{(r)}$ of duration of the diastolic phase, outputting them in step G, of the subject first in the basal condition and then in the perturbed condition.

According to an additional aspect of the invention, the computer-implemented method can further calculate a total power $TP^{(sys)}$ of the power spectrum $PSD_{sys}$ of the resampled diagram $D_{sys}^{(r)}$ of duration of the systolic phase and a total power $TP^{(dia)}$ of the power spectrum $PSD_{dia}$ of the resampled diagram $D_{dia}^{(r)}$ of duration of the diastolic phase, and it can output them in step G, of the subject first in the basal condition and then in the perturbed condition.

It is also specific object of the present invention an apparatus comprising a processing unit configured to execute the computer-implemented method of detecting parameters indicative of a variation of activation of sympathetic nervous system and of a variation of activation of parasympathetic nervous system in a subject in a transition from a basal condition to a perturbed condition as previously described.

It is further specific object of the present invention a set of one or more computer programs comprising instructions which, when executed by one or more processing units, cause said one or more processing units to execute the computer-implemented method of detecting parameters indicative of a variation of activation of sympathetic nervous system and of a variation of activation of parasympathetic nervous system in a subject in a transition from a basal condition to a perturbed condition as previously described.

It is still specific object of the present invention a set of one or more computer-readable media having stored thereon the just described set of one or more computer programs.

The computer-implemented method according to the invention, and the related apparatus, thanks to the separate analysis of the systolic phase and the diastolic phase of the cardiac pressure cycles (such as for instance arterial pressure or pulmonary venous pressure or central venous pressure), allow to provide a reliable indication to identify a variation of activation of the sympathetic nervous system and a variation of activation of the parasympathetic nervous system, as well as to discriminate between an adequate balance and an imbalance between the activation of the sympathetic nervous system and the activation of the parasympathetic nervous system, in a subject in the transition from the basal condition to a perturbed condition. This allows, for example, to determine the effect on the sympathetic and parasympathetic nervous systems of the application of a drug and/or the change in posture of the subject himself/herself.

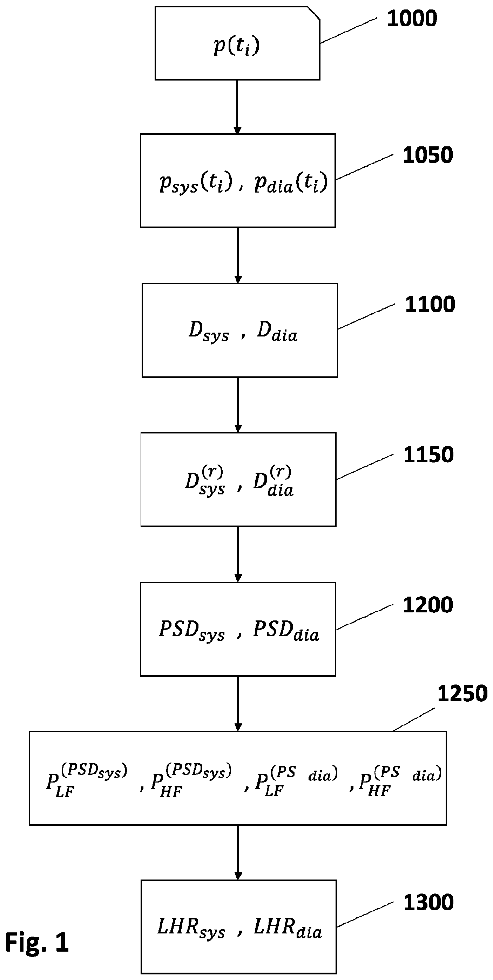

The present invention will be now described, for illustrative but not limiting purposes, according to its preferred embodiments, with particular reference to Figure 1 of the accompanying drawings, that shows a flow graph of the preferred embodiment of the computer-implemented method according to the invention.

The inventor has surprisingly ascertained that, to obtain more information on the activation of the sympathetic nervous system and on the activation of the parasympathetic nervous system, as well as on the balance between them, in a subject on the basis of the variability of the heart rhythm, it is possible to exploit the coupling of heart to the arterial system. To this end, differently from ECG-based detection techniques, the computer-implemented method according to the invention is based on cardiac pressure cycles. The computer-implemented method according to the invention exploits the "mechanical" property of a heartbeat of being composed of two main phases, namely the systolic phase and the diastolic phase.

Differently, prior art methods and apparatuses, employing ECG signal detection, are based on cardiac cycles of electrical signal. Since the electrical signal of a cardiac cycle corresponds only to the electrical component of the electromechanical heart activity that is used to make the blood circulate inside the human body, it is not sufficient to provide all the available information on the balance of the sympathetic and parasympathetic nervous systems, since much of this information is related to the mechanical component of the cardiac activity that actually makes blood circulate. In fact, the electrical signal sent to the heart does not immediately produce a mechanical response of the heart itself, because this also depends on the inertia of the heart and cardiovascular system, i.e. on the specific state of rigidity and compliance of the various systems which form the cardiovascular system. This implies that the prior art methods and apparatuses employing the detection of the ECG signal to evaluate the HRV are affected by errors and approximations which greatly limit their reliability. By way of example, and not by way of limitation, the only electrical component of cardiac activity in the event that the aortic valve does not open correctly, creating problems of electromechanical coupling of the heart to the cardiovascular and respiratory systems, can provide a ECG signal that signals an adequate heartbeat through the detection of the electrical activity of the heart, while instead the mechanical functionality of the heart is strongly compromised and the sympathetic and parasympathetic nervous systems are consequently activated in an unbalanced way with respect to each other; this is applicable in all cases where there is a qualitative dissociation of the electrical component from the mechanical component of the cardiac activity.

The computer-implemented method according to the invention, thanks to the analysis of the variability of the duration separately for the systolic phase and for the diastolic phase of the cardiac cycles of pressure, allows to obtain a detection of parameters indicative of the variations of activation of the sympathetic nervous system and parasympathetic nervous system, from which it is also possible to evaluate a change in the balance between the activity of the sympathetic nervous system and the activity of the parasympathetic nervous system, that allows a reliable evaluation of the activation of the sympathetic and parasympathetic nervous systems and of the degree of their balance. In fact, since the cardiac cycles of pressure have a typical pressure morphology, in which the systolic and diastolic phases are well defined, it is possible to distinguish and hence weigh the contributions to the HRV of the same systolic and diastolic phases and not only of the entire cardiac cycle, as it happens instead for the evaluation of the HRV through the analysis of the tachogram based on the R-R distance of an ECG signal of the prior art methods. In the following the variability of the duration of the systolic phase of the pressure signal will be indicated with SYS-V and the variability of the duration of the diastolic phase of the pressure signal will be indicated with DIA-V.

In particular, the separate analysis of the SYS-V and the DIA-V, carried out by the computer-implemented method according to the invention, can detect parameters indicative of the variations of activation of the sympathetic nervous system and parasympathetic nervous system and of a balance between the activity of the sympathetic nervous system and the activity of the parasympathetic nervous system that is different, in one of or both the systolic and diastolic phases, with respect to the balance evaluated by the detection of the HRV of the entire cardiac cycle based on the ECG signal of the prior art methods.

By way of example, and not by way of limitation, the same HRV could correspond to three different pairs of SYS-V and DIA-V for three respective subjects, namely corresponding to an athlete, a cardiopathic subject, and a normal subject. In fact, in general, even in the case where there are no variations in the R-R distance of an ECG signal in a plurality of cardiac cycles, there can be variations of opposite sign of the duration of the systolic phase and of the duration of the diastolic phase in the same plurality of cardiac cycles due to the adaptation of the organism to specific conditions to which it undergoes. Hence, the variability of these two systolic and diastolic phases can represent contributions of the activation of the sympathetic nervous system and of the activation of the parasympathetic nervous system which are different from each other, whereby the analysis of SYS-V and DIA-V provides more specific information on such contributions. Similarly, in the case where the heart rate of a subject is constant before and after an event, the two mechanical phases that form it, namely the systolic and diastolic phases, can change. In this way, the computer-implemented method according to the invention allows to identify in advance the need or not to cause changes in the contribution of the activation of the sympathetic nervous system with respect to that of the activation of the parasympathetic nervous system, for example through interventions on the component of the activation of the parasympathetic nervous system that affect and change the component of the activation of the sympathetic nervous system, such as the administration of vasoconstrictor or vasodilator or inotropic drugs.

Consequently, the degree of balance of the activation of the sympathetic nervous system and of the activation of the parasympathetic nervous system due to some pathological responses, both to drugs and surgical stresses, that prior art methods based on the ECG signal detection for the HRV measurement fail to identify correctly, is reliably evaluated by the analysis of the SYS-V and the DIA-V carried out by the computer-implemented method according to the invention. In particular, through the analysis of the SYS-V and the DIA-V, the computer-implemented method according to the invention is able to allow to early identify some pathological conditions when they are not yet overt, which the prior art methods identify only after their degeneration.

Figure 1 shows a flow chart of the preferred embodiment of the computer-implemented method according to the invention.

In a first step 1000, the method receives a discrete pressure signal $p(t_i)$ (such as for example an arterial pressure or a pulmonary venous pressure or a central venous pressure) of a subject or patient comprising a plurality of heartbeats. In particular, the discrete pressure signal $p(t_i)$ can derive from a continuous pressure signal $p(t)$ that is detected through pressure sensors and that is digitised to obtain the discrete signal $p(t_i)$ (wherein the index i indicates the succession of discrete samples), or a discrete signal (i.e. already digitised) stored in a memory medium; in particular, the detection of the continuous pressure signal $p(t)$ can take place either invasively or non-invasively, e.g. through a photoplethysmographic sensor. The received discrete pressure signal $p(t_i)$ has a time duration optionally of at least 3 minutes, more optionally of at least 4 minutes, even more optionally of at least 5 minutes.

In a second step 1050, the method identifies each heartbeat of the discrete pressure signal $p(t_i)$ and, within each heartbeat, identifies the systolic phase $p_{sys}(t_i)$ and the diastolic phase $p_{dia}(t_i)$. Optionally, the method performs the identification of each heartbeat through the automated method of discrimination of the heartbeat described in the International application no. WO 2004/084088 A1, and/or the identification of the systolic phase and diastolic phase of each heartbeat on the basis of the identification of the dicrotic notch time (corresponding to the time of closure of the aortic valve for arterial pressure signals or to the time of closure of the tricuspid valve for pulmonary pressure signals).

In a third step 1100, the method builds a diagram $D_{sys}$ of the duration of the systolic phase (ordinate axis) as a function of the progressive number of the heartbeats (abscissa axis) and a diagram $D_{dia}$ of the duration of the diastolic phase (ordinate axis) as a function of the progressive number of the heartbeats (abscissa axis). The duration of the systolic phase and diastolic phase is optionally expressed in milliseconds.

In a fourth step 1150, the method performs a resampling of the diagram $D_{sys}$ of the duration of the systolic phase and of the diagram $D_{dia}$ of the duration of the diastolic phase (built in the third step 1100), obtaining a resampled diagram $D_{sys}^{(r)}$ of the duration of the systolic phase and a resampled diagram $D_{dia}^{(r)}$ of the duration of the diastolic phase.

In a fifth step 1200, the method calculates the power spectrum $PSD_{sys}$ of the resampled diagram $D_{sys}^{(r)}$ of the duration of the systolic phase and the power spectrum $PSD_{dia}$ of the resampled diagram $D_{dia}^{(r)}$ of the duration of the diastolic phase at frequencies between a lower limit frequency $f_{lower\_limit}$, optionally equal to 0,01 Hz, and an upper limit frequency $f_{upper\_limit}$ (higher than the lower limit frequency $f_{lower\_limit}$), optionally variable from 0,4 Hz to 1,2 Hz, more optionally variable from 0,8 Hz to 1,2 Hz, even more optionally equal to 1,2 Hz; in particular, the lower limit frequency $f_{lower\_limit}$ and the upper limit frequency $f_{upper\_limit}$ depend on the type of the subjects under examination. Optionally, the method calculates the power spectra $PSD_{sys}$ and $PSD_{dia}$ through a Fourier transform, more optionally a Fast Fourier Transform (FFT), of the resampled diagram $D_{sys}^{(r)}$ of the duration of the systolic phase and of the resampled diagram $D_{dia}^{(r)}$ of the duration of the diastolic phase, respectively. Alternatively to the Fourier transform, other embodiments of the computer-implemented method according to the invention can calculate the power spectra $PSD_{sys}$ and $PSD_{dia}$ through an autoregressive modelling or through a wavelet transform.

In a sixth step 1250, the method subdivides each of the power spectra $PSD_{sys}$ and $PSD_{dia}$ into three frequency bands VLF (Very Low Frequency), LF (Low Frequency) and HF (High Frequency). In the LF band the frequency $f_{LF}$ ranges from a first intermediate frequency $f_{intermediate\_1}$ to a second intermediate frequency $f_{intermediate\_2}$, thereby

$$f_{intermediate\_1} \leq f_{LF} < f_{intermediate\_2},$$

where the lower limit frequency $f_{lower\_limit}$ is lower than the first intermediate frequency $f_{intermediate\_1}$, that in turn is lower than the second intermediate frequency $f_{intermediate\_2}$, that in turn is lower than the upper limit frequency $f_{upper\_limit}$, thereby

$$f_{lower\_limit} < f_{intermediate\_1} < f_{intermediate\_2} < f_{upper\_limit}.$$

In the HF band the frequency $f_{HF}$ ranges from the second intermediate frequency $f_{intermediate\_2}$ to the upper limit frequency $f_{upper\_limit}$, thereby

$$f_{intermediate\_2} \leq f_{HF} < f_{upper\_limit}.$$

In the VLF band the frequency $f_{VLF}$ ranges from the lower limit frequency $f_{lower\_limit}$ to the first intermediate frequency $f_{intermediate\_1}$ thereby

$$f_{lower\_limit} \leq f_{VLF} < f_{intermediate\_1}.$$

The first intermediate frequency $f_{intermediate\_1}$ and the second intermediate frequency $f_{intermediate\_2}$ also depend on the type of the subjects under examination. Optionally, the first intermediate frequency $f_{intermediate\_1}$ ranges from 0,04 Hz to 0,12 Hz, more optionally it ranges from 0,08 Hz to 0,12 Hz, still more optionally it is equal to 0,12 Hz; optionally, the second intermediate frequency $f_{intermediate\_2}$ ranges from 0,15 Hz to 0,45 Hz, more optionally it ranges from 0,30 Hz to 0,45 Hz, still more optionally it is equal to 0,45 Hz.

Still in the sixth step 1250, the method calculates the power of each of the power spectra $PSD_{sys}$ and $PSD_{dia}$ in each one of the LF and HF bands; namely:

- the power $P_{LF}^{(PSD_{sys})}$ in the LF band of the power spectrum $PSD_{sys}$ (given by the integral in the LF band, i.e. the summation in the discretised domain of frequencies of the power spectrum $PSD_{sys}$);

- the power $P_{HF}^{(PSD_{sys})}$ in the HF band of the power spectrum $PSD_{sys}$ (given by the integral in the HF band, i.e. the summation in the discretised domain of frequencies of the power spectrum $PSD_{sys}$);

- the power $P_{LF}^{(PSD_{dia})}$ in the LF band of the power spectrum $PSD_{dia}$ (given by the integral in the LF band, i.e. the summation in the discretised domain of frequencies of the power spectrum $PSD_{dia}$);

- the power $P_{HF}^{(PSD_{dia})}$ in the HF band of the power spectrum $PSD_{dia}$ (given by the integral in the HF band, i.e. the summation in the discretised domain of frequencies of the power spectrum $PSD_{dia}$).

In a seventh step 1300, the method calculates (and outputs) the values of the ratios $LHR_{sys}$ and $LHR_{dia}$ between the powers in the LF and HF bands of the power spectra $PSD_{sys}$ and $PSD_{dia}$, respectively, thereby:

$$LHR_{sys} = \frac{P_{LF}^{(PSD_{sys})}}{P_{HF}^{(PSD_{sys})}}$$

$$LHR_{dia} = \frac{P_{LF}^{(PSD_{dia})}}{P_{HF}^{(PSD_{dia})}}$$

On the basis of the values of the ratios $LHR_{sys}$ and $LHR_{dia}$ output by the seventh step 1300, taking account of the type of population to which the subject belongs, a doctor is able to evaluate the variation of activation of the sympathetic nervous system and the variation of the activation of the parasympathetic nervous system, from which it is also possible to evaluate a variation in the balance between the activity of the sympathetic nervous system and the activity of the parasympathetic nervous system (i.e., the possible predominance of the activity of the sympathetic nervous system or of the activity of the parasympathetic nervous system on the other) of the subject himself/herself. In particular, the values of the $LHR_{sys}$ and $LHR_{dia}$ ratios depend on the type of population, by age and pathology, to which the examined subjects belong.

In other words, the computer-implemented method according to the invention uses the characteristics of the mechanical response of the cardiovascular system to the electrical stimulus of the heart, analysing the systolic and diastolic phases within each cardiac cycle. This allows for a more reliable evaluation than prior art methods, since the dynamic components of the activations of the sympathetic and parasympathetic nervous system and the balance between the activation of the sympathetic nervous system and the activation of the parasympathetic nervous system give different indications of dynamic equilibrium during the two systolic and diastolic phases, providing more detailed information on stress and vagal activation.

The inventor made some evaluations on the results obtained by applying the computer-implemented method according to the invention and comparing the results with those obtained by the prior art methods in the evaluation of the HRV. In particular, the experiments were conducted on subjects who passed from a basal condition to a perturbed condition in which an event causes a change in the cardiovascular system.

The experiments show that the characteristics of variation of the HRV can be either in agreement or in disagreement with those of the resampled diagram $D_{dia}^{(r)}$ of the duration of the diastolic phase (albeit with non-equal absolute values), and that the characteristics of variation of the resampled diagram $D_{sys}^{(r)}$ of the duration of the systolic phase are often substantially different from those of the HRV.

Some evaluations were carried out on the results obtained for subjects for whom the perturbed condition was caused by the administration of a powerful anaesthetic that has a sympatholytic effect (namely Propofol®). According to traditional physiology, the ratio between the LF and HF components must decrease because the vagal activity is activated and, thus, by inhibiting the activity of the sympathetic nervous system, the balance between the activity of the sympathetic nervous system and the activity of the parasympathetic nervous system changes. However, the characteristics of the HRV had variations that led to conflicting results in the ratio between the LF and HF components of the power spectrum PSD of the tachogram, resulting in a decrease in some subjects and an increase in others, demonstrating that the power spectrum PSD of the tachogram does not correctly identify the activation of the vagal nerve and the inhibition of the sympathetic nervous system. Differently, the ratio $LHR_{sys}$ derived from the resampled diagram $D_{sys}^{(r)}$ of the duration of the systolic phase decreases for all patients, reliably identifying the prevalence of the activation of the parasympathetic nervous system with respect to the basal condition (i.e. to the condition not altered by the administration of the anaesthetic).

In general, the results obtained from the application of the computer-implemented method according to the invention revealed that, to evaluate which one of the sympathetic nervous system and the parasympathetic nervous system is activated in a prevalent way, it is sufficient to carry out a comparison of the variations of the ratios $LHR_{sys}$ and $LHR_{dia}$ in the transition from the basal condition to the perturbed condition in function of the type of subject examined. By way of example, for some types of subjects, if such variations are discordant, the activation of the parasympathetic nervous system has prevailed over the activation of the sympathetic nervous system, while if such variations are in agreement (e.g., both increase), the activation of the sympathetic nervous system has prevailed over the activation of the parasympathetic nervous system.

In the case of a patient under examination (e.g., a patient who has problems of orthostatism, which can lead to syncope, or a patient suffering from liver cirrhosis) and no data related to a basal condition are available, the evaluation of the variation in activation of the sympathetic nervous system and of the variation of the activation of the parasympathetic nervous system, as well as the balance between the activity of the sympathetic nervous system and the activity of the parasympathetic nervous system, are carried out by performing the computer-implemented method according to the invention while the patient is lying down on an examination table, assuming this as the basal condition, subjecting the patient to the so-called tilt test (i.e., the examination table is raised by 60° degrees), assuming this as the perturbed condition, and performing again the computer-implemented method according to the invention. For a patient suffering from liver cirrhosis, it is sufficient to move from a supine position to an orthostatic position as perturbed condition.

It is important to underline that the computer-implemented method according to the invention is not a diagnostic method per se, but it is a method detecting parameters, namely the ratios $LHR_{sys}$ e $LHR_{dia}$, indicative of the variation of activation of the sympathetic nervous system, of the variation of activation of the parasympathetic nervous system, and of a balance between the activity of the sympathetic nervous system and the activity of the parasympathetic nervous system of a subject in the transition from a basal condition to a perturbed condition, which require a subsequent interpretation by a physician for formulating the diagnosis.

Other embodiments of the computer-implemented method according to the invention can also:

- in the second step 1050, identify the value of the dicrotic notch pressure $P_{dic}$ in each heartbeat;
- in the third step 1100, build a diagram $D_{dic}$ of the dicrotic notch pressure (ordinate axis) as a function of the progressive number of the heartbeats (abscissa axis);
- in the fourth step 1150, perform a resampling the diagram $D_{dic}$ of the dicrotic notch pressure obtaining a resampled diagram $D_{dic}^{(r)}$ of the dicrotic notch pressure;
- in the fifth step 1200, calculate the power spectrum $PSD_{dic}$ (optionally through a Fourier transform, more optionally a FFT, or through an autoregressive modelling or through a wavelet transform) of the resampled diagram $D_{dic}^{(r)}$ of the dicrotic notch pressure at frequencies ranging from the lower limit frequency $f_{lower\_limit}$ (optionally equal to 0,01 Hz), and the upper limit frequency $f_{upper\_limit}$ (higher than the lower limit frequency $f_{lower\_limit}$ and optionally ranging from 0,4 Hz to 1,2 Hz, more optionally ranging from 0,8 Hz to 1,2 Hz, still more optionally equal to 1,2 Hz), that, as mentioned, depend on the type of the subjects examined;

- in the sixth step 1250, subdivide the power spectrum $PSD_{dic}$ of the resampled diagram $D_{dic}^{(r)}$ of the dicrotic notch pressure into three frequency bands VLF (in which the frequency $f_{VLF}$ ranges from the lower limit frequency $f_{lower\_limit}$ to the first intermediate frequency $f_{intermediate\_1}$), LF (in which the frequency $f_{LF}$ ranges from the first intermediate frequency $f_{intermediate\_1}$ to the second intermediate frequency $f_{intermediate\_2}$) and HF (in which the frequency $f_{HF}$ ranges from the second intermediate frequency $f_{intermediate\_2}$ to the upper limit frequency $f_{upper\_limit}$), and it calculate, in each of the LF and HF bands, the power of the power spectrum $PSD_{dic}$, namely the power $P_{LF}^{(PSD_{dic})}$ of the power spectrum $PSD_{dic}$ in the LF band (given by the integral in the LF band, i.e. the summation in the discretised domain of the frequencies, of the power spectrum $PSD_{dic}$) and the power $P_{HF}^{(PSD_{dic})}$ of the power spectrum $PSD_{dic}$ in the HF band (given by the integral in the HF band, i.e. the summation in the discretised domain of the frequencies, of the power spectrum $PSD_{dic}$); as mentioned, the first intermediate frequency $f_{intermediate\_1}$ and the second intermediate frequency $f_{intermediate\_2}$ depend on the type of the subjects examined: optionally, the first intermediate frequency $f_{intermediate\_1}$ ranges from 0,04 Hz to 0,12 Hz, more optionally it ranges from 0,08 Hz to 0,12 Hz, still more optionally it is equal to 0,12 Hz; optionally, the second intermediate frequency $f_{intermediate\_2}$ ranges from 0,15 Hz to 0,45 Hz, more optionally it ranges from 0,30 Hz to 0,45 Hz, still more optionally it is equal to 0,45 Hz;

- in the seventh step 1300, calculate (and output) the value of the ratio $LHR_{dic}$ between the powers in the LF and HF bands of the power spectrum $PSD_{dic}$, thereby:

$$LHR_{dic} = \frac{P_{LF}^{(PSD_{dic})}}{P_{HF}^{(PSD_{dic})}}$$

whereby a doctor is able to evaluate the variation of activation of the sympathetic nervous system and the variation of activation of the parasympathetic nervous system as well as the balance between the activity of the sympathetic nervous system and the activity of the parasympathetic nervous system of a subject also on the basis of the value of the ratio $LHR_{dic}$, taking into account the type of population to which the subject belongs.

Further embodiments of the computer-implemented method according to the invention can also determine the HRV according to conventional techniques, whereby:

- in the third step 1100, building a tachogram $D_{beat}$ of the discrete pressure signal $p(t_i)$;
- in the fourth step 1150, performing a resampling of the tachogram $D_{beat}$ of the discrete pressure signal $p(t_i)$ obtaining a resampled tachogram $D_{beat}^{(r)}$ of the discrete pressure signal $p(t_i)$;
- in the fifth step 1200, calculating the power spectrum $PSD_{beat}$ (optionally through a Fourier transform, more optionally a FFT, or through an autoregressive modelling or through a wavelet transform) of the resampled tachogram $D_{beat}^{(r)}$ of the discrete pressure signal $p(t_i)$ at frequencies between 0,01 Hz and 0,4 Hz;

- in the sixth step 1250, subdividing the power spectrum $PSD_{beat}$ of the resampled tachogram $D_{beat}^{(r)}$ of the discrete pressure signal $p(t_i)$ into three frequency bands VLF_HRV (in which the frequency $f_{VLF\_HRV}$ ranges from 0,01 Hz to 0,04 Hz), LF_HRV (in which the frequency $f_{LF\_HRV}$ ranges from 0,04 Hz to 0,15 Hz) e HF_HRV (in which the frequency $f_{HF\_HRV}$ ranges from 0,15 Hz to 0,4 Hz), and calculating, in each one of the bands LF_HRV e HF_HRV, the power of the power spectrum $PSD_{beat}$, namely the power $P_{LF\_HRV}^{(PSD_{beat})}$ in the LF_HRV band of the power spectrum $PSD_{beat}$ (given by the integral in the LF_HRV band, i.e. the summation in the discretised domain of the frequencies, of the power spectrum $PSD_{beat}$) and the power $P_{HF\_HRV}^{(PSD_{beat})}$ in the HF_HRV band of the power spectrum $PSD_{beat}$ (given by the integral in the HF_HRV band, i.e. the summation in the discretised domain of the frequencies, of the power spectrum $PSD_{beat}$); and
- in the seventh phase 1300, calculating (and outputting) the value of the ratio $LHR_{beat}$ between the peak frequencies in the LF_HRV and HF_HRV bands of the power spectrum $PSD_{beat}$, thereby:

$$LHR_{beat} = \frac{P_{LF\_HRV}^{(PSD_{beat})}}{P_{HF\_HRV}^{(PSD_{beat})}}$$

whereby a physician is able to evaluate the variation of activation of the sympathetic nervous system and the variation of activation of the parasympathetic nervous system as well as the balance between the activity of the sympathetic nervous system and the activity of the parasympathetic nervous system of a subject also on the basis of the value of the ratio $LHR_{beat}$, taking into account the type of population to which the subject belongs.

Other embodiments of the computer-implemented method according to the invention can also:

- calculate (optionally in any one of the steps from the fifth step 1200 to the seventh step 1300, and outputting in the seventh step 1300) the standard deviation $SD^{(sys)}$ of the resampled diagram $D_{sys}^{(r)}$ of the duration of the systolic phase and the standard deviation $SD^{(dia)}$ of the resampled diagram $D_{dia}^{(r)}$ of the duration of the diastolic phase (and possibly the standard deviation $SD^{(beat)}$ of the resampled tachogram $D_{beat}^{(r)}$ of the discrete pressure signal $p(t_i)$), and optionally the total power $TP^{(sys)}$ of the power spectrum $PSD_{sys}$ of the resampled diagram $D_{sys}^{(r)}$ of the duration of the systolic phase and the total power $TP^{(dia)}$ of the power spectrum $PSD_{dia}$ of the resampled diagram $D_{dia}^{(r)}$ of the duration of the diastolic phase (and possibly the total power $TP^{(beat)}$ of the power spectrum $PSD_{beat}$ of the resampled tachogram $D_{beat}^{(r)}$ of the discrete pressure signal $p(t_i)$),

whereby a physician, taking into account the type of population to which a subject belongs, is able to evaluate the variation of activation of the sympathetic nervous system and the variation of activation of the parasympathetic nervous system as well as the balance between the activity of the sympathetic nervous system and the activity of the parasympathetic nervous system of the subject himself/herself also on the basis of the value of the standard deviations $SD^{(sys)}$ and $SD^{(dia)}$ (and possibly of the standard deviation $SD^{(beat)}$), and optionally also on the basis of the total powers $TP^{(sys)}$ and $TP^{(dia)}$ (as well as of the total power $TP^{(beat)}$).

Further embodiments of the computer-implemented method according to the invention can build, in the third step 1100, the diagram $D_{sys}$ of the duration of the systolic phase and the diagram $D_{dia}$ of the duration of the diastolic phase expressing the duration of the systolic phase and of the diastolic phase of each heartbeat as a normalised value (e.g., as a percentage value) with respect to the overall heartbeat duration, rather than as an absolute value in milliseconds.

In the above, the preferred embodiments have been described and a number of variations of the present invention have been suggested, but it is to be understood that those skilled in the art can make other variations and changes without departing from the scope of protection thereof, as defined by the appended claims.

**Claims**

1. Computer-implemented method of detecting parameters indicative of a variation of activation of sympathetic nervous system and of a variation of activation of parasympathetic nervous system in a subject in a transition from a basal condition to a perturbed condition, comprising the following steps:

   A. receiving (1000) a discrete pressure signal $p(t_i)$ of the subject comprising a plurality of heartbeats;
   B. identifying (1050) each heartbeat of the discrete pressure signal $p(t_i)$ and, within each heartbeat, identifying a systolic phase $p_{sys}(t_i)$ and a diastolic phase $p_{dia}(t_i)$;
   C. building (1100) a diagram $D_{sys}$ of duration of the systolic phase as a function of a heartbeat progressive number and a diagram $D_{dia}$ of duration of the diastolic phase as a function of the heartbeat progressive number;
   D. executing (1150) a resampling of the diagram $D_{sys}$ of duration of the systolic phase, obtaining a resampled diagram $D_{sys}^{(r)}$ of duration of the systolic phase, and a resampling of the diagram $D_{dia}$ of duration of the diastolic phase, obtaining a resampled diagram $D_{dia}^{(r)}$ of duration of the diastolic phase;
   E. calculating (1200) a power spectrum $PSD_{sys}$ of the resampled diagram $D_{sys}^{(r)}$ of duration of the systolic phase and a power spectrum $PSD_{dia}$ of the resampled diagram $D_{dia}^{(r)}$ of duration of the diastolic phase at frequencies between a lower limit frequency $f_{lower\text{-}limit}$ and a upper limit frequency $f_{upper\_limit}$ higher than the lower limit frequency $f_{lower\_limit}$;
   F. calculating (1250) a power $P_{LF}^{(PSD_{sys})}$ of the power spectrum $PSD_{sys}$ in a LF band, a power $P_{HF}^{(PSD_{sys})}$ of the power spectrum $PSD_{sys}$ in a HF band, a power $P_{LF}^{(PSD_{dia})}$ in the LF band of the power spectrum $PSD_{dia}$, and a power $P_{HF}^{(PSD_{dia})}$ in the HF band of the power spectrum $PSD_{dia}$, wherein the frequency $f_{LF}$ in the LF band is higher than or equal to a first intermediate frequency $f_{intermediate\_1}$ and lower than a second intermediate frequency $f_{intermediate\_2}$, thereby

$$f_{intermediate\_1} \leq f_{LF} < f_{intermediate\_2},$$

wherein the lower limit frequency $f_{lower\_limit}$ is lower than the first intermediate frequency $f_{intermediate\_1}$, that is in turn lower than the second intermediate frequency $f_{intermediate\_2}$, that is in turn lower than the upper limit frequency $f_{upper\_limit}$, thereby

$$f_{lower\_limit} < f_{intermediate\_1} < f_{intermediate\_2} < f_{upper\_limit},$$

and wherein the frequency $f_{HF}$ in the HF band is higher than or equal to the second intermediate frequency $f_{intermediate\_2}$ and lower than the upper limit frequency $f_{upper\_limit}$, thereby

$$f_{intermediate\_2} \leq f_{HF} < f_{upper\_limit};$$

and

G. calculating (1300) and outputting a value of a ratio $LHR_{sys}$ between the powers in the LF and HF bands of the power spectrum $PSD_{sys}$ and a value of a ratio $LHR_{dia}$ between the powers in the LF and HF bands of the power spectrum $PSD_{dia}$, thereby

$$LHR_{sys} = \frac{P_{LF}^{(PSD_{sys})}}{P_{HF}^{(PSD_{sys})}}$$

$$LHR_{dia} = \frac{P_{LF}^{(PSD_{dia})}}{P_{HF}^{(PSD_{dia})}}$$

wherein steps A-G of the computer-implemented method are executed on the subject first in a basal condition and then in a perturbed condition.

2. Computer-implemented method according to claim 1, wherein the lower limit frequency $f_{lower\_limit}$ is equal to 0,01 Hz, the upper limit frequency $f_{upper\_limit}$ ranges from 0,4 Hz to 1,2 Hz, the first intermediate frequency $f_{intermediate\_1}$ ranges from 0,04 Hz to 0,12 Hz, and the second intermediate frequency $f_{intermediate\_2}$ ranges from 0,15 Hz to 0,45 Hz, wherein optionally the upper limit frequency $f_{upper\_limit}$ ranges from 0,8 Hz to 1,2 Hz, the first intermediate frequency $f_{intermediate\_1}$ ranges from 0,08 Hz to 0,12 Hz, and the second intermediate frequency $f_{intermediate\_2}$ ranges from 0,30 Hz to 0,45 Hz, wherein more optionally the upper limit frequency $f_{upper\_limit}$ is equal to 1,2 Hz, the first intermediate frequency $f_{intermediate\_1}$ is equal to 0,12 Hz, and the second intermediate frequency $f_{intermediate\_2}$ is equal to 0,45 Hz.

3. Computer-implemented method according to claim 1 or 2, wherein, in step E, the power spectra $PSD_{sys}$ and $PSD_{dia}$ are calculated through a Fourier transform, optionally through a Fast Fourier Transform (FFT), of the resampled diagram $D_{sys}^{(r)}$ of duration of the systolic phase and of the resampled diagram $D_{dia}^{(r)}$ of duration of the diastolic phase, respectively.

4. Computer-implemented method according to any one of the preceding claims, wherein the discrete pressure signal $p(t_i)$ received in step A has a time duration of at least 3 minutes, optionally of at least 4 minutes, more optionally of at least 5 minutes.

5. Computer-implemented method according to any one of the preceding claims, wherein, in step B, the systolic phase and the diastolic phase of each heartbeat are identified on the basis of identification of the dicrotic notch time.

6. Computer-implemented method according to claim 5, that:

- in step B, further identifies a value of dicrotic notch pressure $P_{dic}$ in each heartbeat;
- in step C, further builds a diagram $D_{dic}$ of dicrotic notch pressure as a function of the heartbeat progressive number;
- in step D, further executes a resampling of the diagram $D_{dic}$ of dicrotic notch pressure obtaining a resampled diagram $D_{dic}^{(r)}$ of dicrotic notch pressure;

- in step E, further calculates a power spectrum $PSD_{dic}$ of the resampled diagram $D_{dic}^{(r)}$ of dicrotic notch pressure at frequencies between the lower limit frequency $f_{lower\_limit}$ and the upper limit frequency $f_{upper\_limit}$;
- in step F, further calculates a power $P_{LF}^{(PSD_{dic})}$ of the power spectrum $PSD_{dic}$ in the LF band and a power $P_{HF}^{(PSD_{dic})}$ of the power spectrum $PSD_{dic}$ in the HF band; and
- in step G, further calculates and outputs a value of a ratio $LHR_{dic}$ between the powers in the LF and HF bands of the power spectrum $PSD_{dic}$, thereby:

$$LHR_{dic} = \frac{P_{LF}^{(PSD_{dic})}}{P_{HF}^{(PSD_{dic})}}$$

7. Computer-implemented method according to any one of the preceding claims, wherein in step C the diagram $D_{sys}$ of duration of the systolic phase and the diagram $D_{dia}$ of duration of the diastolic phase are built by expressing the duration of the systolic phase and of the diastolic phase of each heartbeat as value normalised to an overall duration of the heartbeat under consideration.

8. Computer-implemented method according to any one of the preceding claims, further comprising determining and outputting a HRV (*Heart Rate Variability*) of the subject first in the basal condition and then in the perturbed condition.

9. Computer-implemented method according to any one of the preceding claims, that further calculates a standard deviation $SD^{(sys)}$ of the resampled diagram $D_{sys}^{(r)}$ of duration of the systolic phase and a standard deviation $SD^{(dia)}$ of the resampled diagram $D_{dia}^{(r)}$ of duration of the diastolic phase, outputting them in step G, of the subject first in the basal condition and then in the perturbed condition.

10. Computer-implemented method according to any one of the preceding claims, that further calculates a total power $TP^{(sys)}$ of the power spectrum $PSD_{sys}$ of the resampled diagram $D_{sys}^{(r)}$ of duration of the systolic phase and a total power $TP^{(dia)}$ of the power spectrum $PSD_{dia}$ of the resampled diagram $D_{dia}^{(r)}$ of duration of the diastolic phase, outputting them in step G, of the subject first in the basal condition and then in the perturbed condition.

11. Apparatus comprising a processing unit configured to execute the computer-implemented method of detecting parameters indicative of a variation of activation of sympathetic nervous system and of a variation of activation of parasympathetic nervous system in a subject in a transition from a basal condition to a perturbed condition according to any one of claims 1-10.

12. Set of one or more computer programs comprising instructions which, when executed by one or more processing units, cause said one or more processing units to execute the computer-implemented method of detecting parameters indicative of a variation of activation of sympathetic nervous system and of a variation of activation of parasympathetic nervous system in a subject in a transition from a basal condition to a perturbed condition according to any one of claims 1-10.

13. Set of one or more computer-readable media having stored thereon the set of one or more computer programs according to claim 12.

**Patentansprüche**

1. Computerimplementiertes Verfahren zum Erfassen von Parametern, die eine Veränderung der Aktivierung des sympathischen Nervensystems und eine Veränderung der Aktivierung des parasympathischen Nervensystems bei einem Subjekt in einem Übergang von einem Grundzustand zu einem gestörten Zustand anzeigen, umfassend die folgenden Schritte:

> A. Empfang (1000) eines diskreten Drucksignals $p(t_i)$ des Patienten, das eine Vielzahl von Herzschlägen umfasst;
>
> B. Identifizierung (1050) jedes Herzschlags des diskreten Drucksignals $p(t_i)$ und, innerhalb jedes Herzschlags, Identifizierung einer systolischen Phase $p_{sys}(t_i)$ und einer diastolischen Phase; $p_{dia}(t_i)$
>
> C. Erstellung (1100) eines Diagramms $D_{sys}$ der Dauer der systolischen Phase in Abhängigkeit von einer Herzschlag-Progressionszahl und eines Diagramms $D_{dia}$ der Dauer der diastolischen Phase in Abhängigkeit von der Herzschlag-Progressionszahl;
>
> D. Ausführen (1150) einer Neuabtastung des Diagramms $D_{sys}$ der Dauer der systolischen Phase, wodurch ein neu abgetastetes Diagramm $D_{sys}^{(r)}$ der Dauer der systolischen Phase erhalten wird, und einer Neuabtastung des Diagramms $D_{dia}$ der Dauer der diastolischen Phase, wodurch ein neu abgetastetes Diagramm $D_{dia}^{(r)}$ der Dauer der diastolischen Phase erhalten wird;
>
> E. Berechnen (1200) eines Leistungsspektrums $PSD_{sys}$ des neu abgetasteten Diagramms $D_{sys}^{(r)}$ der Dauer der systolischen Phase und eines Leistungsspektrums $PSD_{dia}$ des neu abgetasteten Diagramms $D_{dia}^{(r)}$ der Dauer der diastolischen Phase bei Frequenzen zwischen einer unteren Grenzfrequenz $f_{lower\text{-}limit}$ und einer oberen Grenzfrequenz $f_{upper\_limit}$, die höher ist als die untere Grenzfrequenz $f_{lower\_limit}$;
>
> F. Berechnen (1250) einer Leistung $P_{LF}^{(PSD_{sys})}$ des Leistungsspektrums $PSD_{sys}$ in einem NF-Band, einer Leistung $P_{HF}^{(PSD_{sys})}$ des Leistungsspektrums $PSD_{sys}$ in einem HF-Band, einer Leistung $P_{LF}^{(PSD_{dia})}$ im NF-Band des Leistungsspektrums $PSD_{dia}$ und einer Leistung $P_{HF}^{(PSD_{dia})}$ im HF-Band des Leistungsspektrums $PSD_{dia}$, wobei die Frequenz $f_{LF}$ im NF-Band höher als oder gleich einer ersten Zwischenfrequenz $f_{intermediate\_1}$ und niedriger als eine zweite Zwischenfrequenz $f_{intermediate\_2}$ ist, wodurch
>
> $$f_{intermediate\_1} \le f_{LF} < f_{intermediate\_2},$$
>
> wobei die untere Grenzfrequenz $f_{lower\_limit}$ niedriger ist als die erste Zwischenfrequenz $f_{intermediate\_1}$, die ihrerseits niedriger ist als die zweite Zwischenfrequenz $f_{intermediate\_2}$, die ihrerseits niedriger ist als die obere Grenzfrequenz $f_{upper\_limit}$, wodurch
>
> $$f_{lower\_limit} < f_{intermediate\_1} < f_{intermediate\_2} < f_{upper\_limit},$$
>
> und wobei die Frequenz $f_{HF}$ im HF-Band höher als oder gleich der zweiten Zwischenfrequenz $f_{intermediate\_2}$ und niedriger als die obere Grenzfrequenz $f_{upper\_limit}$ ist, wodurch
>
> $$f_{intermediate\_2} \le f_{HF} < f_{upper\_limit}$$
>
> und
>
> G. Berechnen (1300) und Ausgeben eines Wertes eines Verhältnisses $LHR_{sys}$ zwischen den Leistungen im NF- und HF-Band des Leistungsspektrums $PSD_{sys}$ und eines Wertes eines Verhältnisses $LHR_{dia}$ zwischen den Leistungen im NF- und HF-Band des Leistungsspektrums $PSD_{dia}$, wodurch

$$LHR_{sys} = \frac{P_{LF}^{(PSD_{sys})}}{P_{HF}^{(PSD_{sys})}}$$

$$LHR_{dia} = \frac{P_{LF}^{(PSD_{dia})}}{P_{HF}^{(PSD_{dia})}}$$

wobei die Schritte A-G des computerimplementierten Verfahrens an dem Subjekt zunächst in einem Grundzustand und dann in einem gestörten Zustand ausgeführt werden.

2. Computerimplementiertes Verfahren nach Anspruch 1, wobei die untere Grenzfrequenz $f_{lower\_limit}$ gleich 0,01 Hz ist, die obere Grenzfrequenz $f_{upper\_limit}$ im Bereich von 0,4 Hz bis 1,2 Hz liegt, die erste Zwischenfrequenz $f_{intermediate\_1}$ im Bereich von 0,04 Hz bis 0,12 Hz liegt, und die zweite Zwischenfrequenz $f_{intermediate\_2}$ im Bereich von 0,15 Hz bis 0,45 Hz liegt, wobei optional die obere Grenzfrequenz $f_{upper\_limit}$ im Bereich von 0,8 Hz bis 1,2 Hz, die erste Zwischenfrequenz $f_{intermediate\_1}$ im Bereich von 0,08 Hz bis 0,12 Hz und die zweite Zwischenfrequenz $f_{intermediate\_2}$ im Bereich von 0,30 Hz bis 0,45 Hz liegt, wobei optional die obere Grenzfrequenz $f_{upper\_limit}$ gleich 1,2 Hz, die erste Zwischenfrequenz $f_{intermediate\_1}$ gleich 0,12 Hz und die zweite Zwischenfrequenz $f_{intermediate\_2}$ gleich 0,45 Hz ist.

3. Computerimplementiertes Verfahren nach Anspruch 1 oder 2, wobei in Schritt E die Leistungsspektren $PSD_{sys}$ und $PSD_{dia}$ durch eine Fouriertransformation, gegebenenfalls durch eine schnelle Fouriertransformation (FFT), des neu abgetasteten Diagramms $D_{sys}^{(r)}$ der Dauer der systolischen Phase bzw. des neu abgetasteten Diagramms $D_{dia}^{(r)}$ der Dauer der diastolischen Phase berechnet werden.

4. Computerimplementiertes Verfahren nach einem der vorhergehenden Ansprüche, wobei das in Schritt A empfangene diskrete Drucksignal $p(t_i)$ eine Zeitdauer von mindestens 3 Minuten, optional von mindestens 4 Minuten, optional von mindestens 5 Minuten hat.

5. Computerimplementiertes Verfahren nach einem der vorhergehenden Ansprüche, wobei in Schritt B die systolische Phase und die diastolische Phase jedes Herzschlags auf der Grundlage der Identifizierung der dicrotic notch Zeit identifiziert werden.

6. Computerimplementiertes Verfahren nach Anspruch 5, das:

- in Schritt B ferner einen Wert des dikrotischen Kerbdrucks $P_{dic}$ bei jedem Herzschlag ermittelt;
- in Schritt C ein Diagramm $D_{dic}$ des dikrotischen Kerbdrucks als Funktion der progressiven Herzschlagzahl erstellt;
- in Schritt D eine Neuabtastung des Diagramms $D_{dic}$ des dikrotischen Kerbdrucks durchführt, um ein neu abgetastetes Diagramm $D_{dic}^{(r)}$ des dikrotischen Kerbdrucks zu erhalten;

- In Schritt E ferner ein Leistungsspektrum $PSD_{dic}$ des neu abgetasteten Diagramms $D_{dic}^{(r)}$ des dikrotischen Kerbdrucks bei Frequenzen zwischen der unteren Grenzfrequenz $f_{lower-limit}$ und der oberen Grenzfrequenz $f_{upper\_limit}$ berechnet;

- in Schritt F ferner eine Leistung $P_{LF}^{(PSD_{dic})}$ des Leistungsspektrums $PSD_{dic}$ im LF-Band und eine Leistung $P_{HF}^{(PSD_{dic})}$ des Leistungsspektrums $PSD_{dic}$ im HF-Band berechnet; und
- in Schritt G ferner einen Wert eines Verhältnisses $LHR_{dic}$ zwischen den Leistungen im NF- und HF-Band des Leistungsspektrums $PSD_{dic}$ berechnet und ausgibt, sodass:

$$LHR_{dic} = \frac{P_{LF}^{(PSD_{dic})}}{P_{HF}^{(PSD_{dic})}}$$

7. Computerimplementiertes Verfahren nach einem der vorhergehenden Ansprüche, wobei in Schritt C das Diagramm $D_{sys}$ der Dauer der systolischen Phase und das Diagramm $D_{dia}$ der Dauer der diastolischen Phase erstellt werden, indem die Dauer der systolischen Phase und der diastolischen Phase jedes Herzschlags als Wert ausgedrückt wird, der auf eine Gesamtdauer des betrachteten Herzschlags normiert ist.

8. Computerimplementiertes Verfahren nach einem der vorangehenden Ansprüche, das ferner die Bestimmung und Ausgabe einer HRV (*Herzfrequenzvariabilität*) des Probanden zunächst im Grundzustand und dann im gestörten Zustand umfasst.

9. Computerimplementiertes Verfahren nach einem der vorhergehenden Ansprüche, das ferner eine Standardabweichung $SD^{(sys)}$ des neu abgetasteten Diagramms $D_{sys}^{(r)}$ der Dauer der systolischen Phase und eine Standardabweichung $SD^{(dia)}$ des neu abgetasteten Diagramms $D_{dia}^{(r)}$ der Dauer der diastolischen Phase berechnet und sie in Schritt G ausgibt, und zwar für das Subjekt zunächst im Grundzustand und dann im gestörten Zustand.

10. Computerimplementiertes Verfahren nach einem der vorhergehenden Ansprüche, das ferner eine Gesamtleistung $TP^{(sys)}$ des Leistungsspektrums $PSD_{sys}$ des neu abgetasteten Diagramms $D_{sys}^{(r)}$ der Dauer der systolischen Phase und eine Gesamtleistung $TP^{(dia)}$ des Leistungsspektrums $PSD_{dia}$ des neu abgetasteten Diagramms $D_{dia}^{(r)}$ der Dauer der diastolischen Phase berechnet und sie in Schritt G ausgibt, und zwar für das Subjekt zunächst im Grundzustand und dann im gestörten Zustand.

11. Vorrichtung, die eine Verarbeitungseinheit umfasst, die so konfiguriert ist, dass sie das computerimplementierte Verfahren zum Erfassen von Parametern ausführt, die eine Veränderung der Aktivierung des sympathischen Nervensystems und eine Veränderung der Aktivierung des parasympathischen Nervensystems bei einem Subjekt beim Übergang von einem Grundzustand zu einem gestörten Zustand nach einem der Ansprüche 1-10 anzeigen.

12. Satz von einem oder mehreren Computerprogrammen mit Befehlen, die, wenn sie von einer oder mehreren Verarbeitungseinheiten ausgeführt werden, die eine oder mehrere Verarbeitungseinheiten veranlassen, das computerimplementierte Verfahren zum Erfassen von Parametern auszuführen, die eine Variation der Aktivierung des sympathischen Nervensystems und eine Variation der Aktivierung des parasympathischen Nervensystems bei einem Subjekt in einem Übergang von einem Grundzustand zu einem gestörten Zustand nach einem der Ansprüche 1-10 anzeigen.

13. Satz von einem oder mehreren computerlesbaren Medien, auf denen der Satz von einem oder mehreren Computerprogrammen nach Anspruch 12 gespeichert ist.

**Revendications**

1. Procédé mis en œuvre par ordinateur de détection de paramètres indiquant une variation d'activation du système nerveux sympathique et une variation d'activation du système nerveux parasympathique chez un sujet lors d'un passage d'un état basal à un état perturbé, comprenant les étapes suivantes :

A. la réception (1000) d'un signal de pression discret $p(t_i)$ du sujet comprenant une pluralité de battements cardiaques ;
B. l'identification (1050) de chaque battement cardiaque du signal de pression discret $p(t_i)$ et, dans chaque battement cardiaque, l'identification d'une phase systolique $p_{sys}(t_i)$ et d'une phase diastolique $p_{dia}(t_i)$ ;
C. la construction (1100) d'un diagramme $D_{sys}$ de durée de la phase systolique en fonction d'un nombre progressif de battements cardiaques et d'un diagramme $D_{dia}$ de durée de la phase diastolique en fonction du nombre progressif de battements cardiaques ;

D. l'exécution (1150) d'un rééchantillonnage du diagramme $D_{sys}$ de durée de la phase systolique, obtenant un diagramme rééchantillonné $D_{sys}^{(r)}$ de durée de la phase systolique, et d'un rééchantillonnage du diagramme $D_{dia}$ de durée de la phase diastolique, obtenant un diagramme rééchantillonné $D_{dia}^{(r)}$ de durée de la phase diastolique ;

E. le calcul (1200) d'un spectre de puissance $PSD_{sys}$ du diagramme rééchantillonné $D_{sys}^{(r)}$ de la phase systolique et d'un spectre de puissance $PSD_{dia}$ du diagramme rééchantillonné $D_{dia}^{(r)}$ de durée de la phase diastolique à des fréquences comprises entre une fréquence limite inférieure $f_{lower\_limit}$ et une fréquence limite supérieure $f_{upper\_limit}$ supérieure à la fréquence limite inférieure $f_{lower\_limit}$ ;

F. le calcul (1250) d'une puissance $P_{LF}^{(PSD_{sys})}$ du spectre de puissance $PSD_{sys}$ dans une bande LF, d'une puissance $P_{HF}^{(PSD_{sys})}$ du spectre de puissance $PSD_{sys}$ dans une bande HF, d'une puissance $P_{LF}^{(PSD_{dia})}$ dans la bande LF du spectre de puissance $PSD_{dia}$, et d'une puissance $P_{HF}^{(PSD_{dia})}$ dans la bande HF du spectre de puissance $PSD_{dia}$, où la fréquence $f_{LF}$ dans la bande LF est supérieure ou égale à une première fréquence intermédiaire $f_{intermediate\_1}$ et inférieure à une seconde fréquence intermédiaire $f_{intermediate\_2}$, de ce fait

$$f_{intermediate\_1} \leq f_{LF} < f_{intermediate\_2},$$

où la fréquence limite inférieure $f_{lower\_limit}$ est inférieure à la première fréquence intermédiaire $f_{intermediate\_1}$, qui est à son tour inférieure à la seconde fréquence intermédiaire $f_{intermediate\_2}$ qui est à son tour inférieure à la fréquence limite supérieure $f_{upper\_limit}$, de ce fait

$$f_{lower\_limit} < f_{intermediate\_1} < f_{intermediate\_2} < f_{upper\_limit}$$

et où la fréquence $f_{HF}$ dans la bande HF est supérieure ou égale à la seconde fréquence intermédiaire $f_{intermediate\_2}$ et inférieure à la fréquence limite supérieure $f_{upper\_limit}$, de ce fait

$$f_{intermediate\_2} \leq f_{HF} < f_{upper\_limit} ;$$

et

G. le calcul (1300) et la sortie d'une valeur d'un rapport $LHR_{sys}$ entre les puissances dans les bandes LF et HF du spectre de puissance $PSD_{sys}$ et d'une valeur d'un rapport $LHR_{dia}$ entre les puissances dans les bandes LF et HF du spectre de puissance $PSD_{dia}$, de ce fait

$$LHR_{sys} = \frac{P_{LF}^{(PSD_{sys})}}{P_{HF}^{(PSD_{sys})}}$$

$$LHR_{dia} = \frac{P_{LF}^{(PSD_{dia})}}{P_{HF}^{(PSD_{dia})}}$$

où les étapes A à G du procédé mis en œuvre par ordinateur sont exécutées sur le sujet d'abord dans un état basal, puis dans un état perturbé.

2. Procédé mis en œuvre par ordinateur selon la revendication 1, dans lequel la fréquence limite inférieure $f_{lower\_limit}$ est égale à 0,01 Hz, la fréquence limite supérieure $f_{upper\_limit}$ est comprise entre 0,4 Hz et 1,2 Hz, la première fréquence intermédiaire $f_{intermediate\_1}$ est comprise entre 0,04 Hz et 0,12 Hz, et la seconde fréquence intermédiaire $f_{intermediate\_2}$

est comprise entre 0,15 Hz et 0,45 Hz, où éventuellement la fréquence limite supérieure $f_{upper\_limit}$ est comprise entre 0,8 Hz et 1,2 Hz, la première fréquence intermédiaire $f_{intermediate\_1}$ est comprise entre 0,08 Hz et 0,12 Hz, et la seconde fréquence intermédiaire $f_{intermediate\_2}$ est comprise entre 0,30 Hz et 0,45 Hz, où plus éventuellement la fréquence limite supérieure $f_{upper\_limit}$ est égale à 1,2 Hz, la première fréquence intermédiaire $f_{intermediate\_1}$ est égale à 0,12 Hz, et la seconde fréquence intermédiaire $f_{intermediate\_2}$ est égale à 0,45 Hz.

3. Procédé mis en œuvre par ordinateur selon la revendication 1 ou 2, dans lequel, à l'étape E, les spectres de puissance $PSD_{sys}$ et $PSD_{dia}$ sont calculés par une transformation de Fourier, éventuellement par une transformation de Fourier rapide (FFT), du diagramme rééchantillonné $D_{sys}^{(r)}$ de durée de la phase systolique et du diagramme rééchantillonné $D_{dia}^{(r)}$ de durée de la phase diastolique, respectivement.

4. Procédé mis en œuvre par ordinateur selon l'une quelconque des revendications précédentes, dans lequel le signal de pression discret $p(t_i)$ reçu à l'étape A a une durée d'au moins 3 minutes, éventuellement d'au moins 4 minutes, plus éventuellement d'au moins 5 minutes.

5. Procédé mis en œuvre par ordinateur selon l'une quelconque des revendications précédentes, dans lequel, à l'étape B, la phase systolique et la phase diastolique de chaque battement cardiaque sont identifiées sur la base de l'identification du temps d'incisure dicrote.

6. Procédé mis en œuvre par ordinateur selon la revendication 5, qui :

   - à l'étape B, identifie en outre une valeur de pression d'incisure dicrote $P_{dic}$ dans chaque battement cardiaque ;
   - à l'étape C, construit en outre un diagramme $D_{dic}$ de pression d'incisure dicrote en fonction du nombre progressif de battements cardiaques ;
   - à l'étape D, exécute en outre un rééchantillonnage du diagramme $D_{dic}$ de pression d'incisure dicrote obtenant un diagramme rééchantillonné $D_{dic}^{(r)}$ de pression d'incisure dicrote ;

   - à l'étape E, calcule en outre un spectre de puissance $PSD_{dic}$ du diagramme rééchantillonné $D_{dic}^{(r)}$ de pression d'incisure dicrote à des fréquences comprises entre la fréquence limite inférieure $f_{lower\_limit}$ et la fréquence limite supérieure $f_{upper\_limit}$;
   - à l'étape F, calcule en outre une puissance $P_{LF}^{(PSD_{dic})}$ du spectre de puissance $PSD_{dic}$ dans la bande LF et une puissance $P_{HF}^{(PSD_{dic})}$ du spectre de puissance $PSD_{dic}$ dans la bande HF ; et
   - à l'étape G, calcule en outre et délivre en sortie une valeur d'un rapport $LHR_{dic}$ entre les puissances dans les bandes LF et HF du spectre de puissance $PSD_{dic}$, de ce fait :

$$LHR_{dic} = \frac{P_{LF}^{(PSD_{dic})}}{P_{HF}^{(PSD_{dic})}}$$

7. Procédé mis en œuvre par ordinateur selon l'une quelconque des revendications précédentes, dans lequel à l'étape C le diagramme $D_{sys}$ de durée de la phase systolique et le diagramme $D_{dia}$ de durée de la phase diastolique sont construits en exprimant la durée de la phase systolique et de la phase diastolique de chaque battement cardiaque comme valeur normalisée par rapport à une durée globale du battement cardiaque considéré.

8. Procédé mis en œuvre par ordinateur selon l'une quelconque des revendications précédentes, comprenant en outre la détermination et la sortie d'une HRV (*variabilité de fréquence cardiaque*) du sujet d'abord dans l'état basal puis dans l'état perturbé.

9. Procédé mis en œuvre par ordinateur selon l'une quelconque des revendications précédentes, qui calcule en outre un écart type $SD^{(sys)}$ du diagramme rééchantillonné $D_{sys}^{(r)}$ de durée de la phase systolique et un écart type $SD^{(dia)}$ du diagramme rééchantillonné $D_{dia}^{(r)}$ de durée de la phase diastolique, les délivrant en sortie à l'étape G, du sujet d'abord dans l'état basal puis dans l'état perturbé.

10. Procédé mis en œuvre par ordinateur selon l'une quelconque des revendications précédentes, qui calcule en outre une puissance totale $TP^{(sys)}$ du spectre de puissance $PSD_{sys}$ du diagramme rééchantillonné $D_{sys}^{(r)}$ de durée de la phase systolique et une puissance totale $TP^{(dia)}$ du spectre de puissance $PSD_{dia}$ du diagramme rééchantillonné $D_{dia}^{(r)}$ de durée de la phase diastolique, les délivrant à l'étape G, du sujet d'abord dans l'état basal puis dans l'état perturbé.

11. Appareil comprenant une unité de traitement configurée pour exécuter le procédé mis en œuvre par ordinateur de détection de paramètres indiquant une variation d'activation du système nerveux sympathique et une variation d'activation du système nerveux parasympathique chez un sujet lors d'un passage d'un état basal à un état perturbé selon l'une quelconque des revendications 1 à 10.

12. Ensemble d'un ou de plusieurs programmes informatiques comprenant des instructions qui, lorsqu'elles sont exécutées par une ou plusieurs unités de traitement, amènent lesdites une ou plusieurs unités de traitement à exécuter le procédé mis en œuvre par ordinateur de détection de paramètres indiquant une variation d'activation du système nerveux sympathique et une variation d'activation du système nerveux parasympathique chez un sujet lors d'un passage d'un état basal à un état perturbé selon l'une quelconque des revendications 1 à 10.

13. Ensemble d'un ou de plusieurs supports lisibles par ordinateur sur lesquels est stocké l'ensemble d'un ou de plusieurs programmes informatiques selon la revendication 12.

$p(t_i)$ — 1000

$p_{sys}(t_i)$ , $p_{dia}(t_i)$ — 1050

$D_{sys}$ , $D_{dia}$ — 1100

$D_{sys}^{(r)}$ , $D_{dia}^{(r)}$ — 1150

$PSD_{sys}$ , $PSD_{dia}$ — 1200

1250

$P_{LF}^{(PSD_{sys})}$ , $P_{HF}^{(PSD_{sys})}$ , $P_{LF}^{(PS\ dia)}$ , $P_{HF}^{(PS\ dia)}$

$LHR_{sys}$ , $LHR_{dia}$ — 1300

**Fig. 1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004084088 A1

**Non-patent literature cited in the description**

- **J. W. HURST,**. Naming of the Waves in the ECG, With a Brief Account of Their Genesis. *Circulation*, 03 November 1998, vol. 98 (18), 1937-42
- **J. PUCIK et al.** Heart Rate Variability Spectrum: Physiologic Aliasing and Nonstationarity Considerations. Trends in Biomedical Engineering Conference paper, 16 September 2009
- **A.E. AUBERT et al.** Heart rate variability in athletes. *Sports Medicine*, 2003, vol. 33 (12), 889-919
- **CHENGYU LIU et al.** Systolic and Diastolic Time Interval Variability Analysis and Their Relations with Heart Rate Variability. BIOINFORMATICS AND BIOMEDICAL ENGINEERING, 2009
- 3RD INTERNATIONAL CONFERENCE ON. ICBBE 2009. IEEE, 11 June 2009, 1-4
- Respiratory variation of systolic and diastolic time intervals within radial arterial waveform: a comparison with dynamic preload index. **PARK JI HYUN et al.** JOURNAL OF CLINICAL ANESTHESIA. BUTTERWORTH PUBLISHERS, 24 March 2016, vol. 32, 75-81